# EUROPÄISCHE PATENTSCHRIFT

(11) **EP 1 894 995 B1**
(45) Veröffentlichungstag und Bekanntmachung des Hinweises auf die Patenterteilung: **27.10.2010**
(21) Anmeldenummer: 05749360.3
(22) Anmeldetag: 28.04.2005
(51) Int. Cl.: C12N 1/20, A61K 35/66, C12R 1/07

(54) **IRILIS-BIOPRÄPARATION AUF DER BASIS VON DARIN ENTHALTENEN BACILLUS-BAKTERIENSTÄMMEN BACILLUS SUBTILIS UND BACILLUS LICHENIFORMIS**
IRILIS BIOPREPARATION BASED ON BACILLUS-STRAIN BACTERIA, BACILLUS SUBTILIS AND BACILLUS LICHENIFORMIS CONTAINED THEREIN
PRÉPARATION BIOLOGIQUE IRILIS À BASE DE BACTÉRIES DU GENRE BACILLUS CONTENANT BACILLUS SUBTILIS ET BACILLUS LICHENIFORMIS

(43) Veröffentlichungstag der Anmeldung: 05.03.2008
(73) Patentinhaber: Vasilieva, Elena Aleksandrovna, Moscow 117292 (RU); Osipova, Irina Grigorievna, Moscow 125171 (RU); Sorokulova, Irina Borisovna, Kiev, 252005 (UA)
(72) Erfinder: TROFIMOV, Sergei Gennadievich, Chelyabinsk (RU); EVLASHKINA, Vera Franzevna, Moscow, 121355 (RU); HARITONOVA, Elena Yurievna, Moscow, 121309 (RU); SARKISOV, Sergei Eduardovich, Moscow, 103009 (RU); TERESHKINA, Natalia Vasilievna, Moscow, 123479 (RU); OSTROUMOV, Yurii Igorevich, Moscow, 117405 (RU); DORONIN, Aleksandr Nikolaevich, Ekaterinburg, 620048 (RU); KULICHIZKAYA, Marina Aleksandrovna, Nizhny Novgorod, 603086 (RU); MATVEEV, Aleksandr Aleksandrovich, Moscow, 117449 (RU)
(74) Vertreter: Jeck, Anton
(86) Internationale Anmeldenummer: PCT/RU2005/000231
(87) Internationale Veröffentlichungsnummer: WO 2006/115430

(56) Entgegenhaltungen:
- EP-A- 1 031 287
- EP-A- 1 472 933
- WO-A-2005/019417
- RU-C1- 2 132 196
- RU-C1- 2 142 287
- RU-C1- 2 159 625
- RU-C1- 2 182 009
- RU-C1- 2 219 238
- US-B1- 6 723 326
- SANDERS M E ET AL: "Sporeformers as human probiotics: Bacillus, Sporolactobacillus, and Brevibacillus" COMPREHENSIVE REVIEWS IN FOOD SCIENCE AND FOOD SAFETY, INSTITUTE OF FOOD TECHNOLOGISTS, CHICAGO, IL, US, Bd. 2, 1. Januar 2003 (2003-01-01), Seiten 101-110, XP002412617 ISSN: 1541-4337
- OSIPOVA I G ET AL: "Pre-clinical trials of new spore probiotics" VESTNIK ROSSIISKOI AKADEMII MEDITSINSKIKH NAUK, Nr. 12, 2005, Seiten 36-40, XP009109797 ISSN: 0869-6047

## Beschreibung

Die Entwicklung gehört zum Bereich der Biotechnologie und bezieht sich auf die Entwicklung der neuen probiotischen Präparate auf Basis der Bakterien der Gattung *Bacillus,* die für die Prophylaxe und Behandlung der Infektionserkrankungen und Dysbiose unterschiedlicher Krankheitsursachen des Menschen, des Viehs und Geflügels verwendet werden können.

Biopräparate auf Basis der Bakterien der Gattung *Bacillus* sind gut bekannt. Zur Zusammensetzung der Biopräparate-Probiotik auf Basis der Bakterien der Gattung Bacillus gehören Stämme, die über ein unterschiedliches Spektrum der antibakteriellen und antimykotischen Aktivität verfügen. Die Effizienz der Präparate wird durch das breite Spektrum und Wirkung der antibakteriellen Aktivität der im Präparat beinhalteten Stämme bestimmt.

Eine weite Verbreitung in medizinischer Praxis haben Probiotikum Bakgisubtil (Firma "Marion Merrell" /Frankreich/) und sein Analogon Flonivin BS (Firma "Galenika" /Jugoslawien/) in westeuropäischen Ländern bekommen, die die Kultur des Stammes *B.sereus* IP 5832 (Sammlung des Paster-Instituts (Paris) beinhalten. Bekannt ist das vietnamesische Präparat Biosubtil, das den Stamm *B.subtilis* als wirksamen Stoff beinhaltet. Die Basis von Enterogermin (Firma "Sanofi Winthrop", Italien) ist die Kultur *B. clausii.* Bekannt ist auch das Biopräparat Cereobigen ("Xing Lap", China), dessen Wirkstoff der Stamm *B.cerius* DM-423 ist (W.W.Smirnow, S.R. Resnik, 1.B.Sorokulowa, W.A.Wjunitskaja. Dissussionsfragen der Entwicklung und Anwendung der bakteriellen Präparate für die Korrektur der Mikroflora der warmblütigen Tiere// Mikrobiologie Zeitschrift, 1992, Band 54, Nr., S. 82-93).

Der Mangel dieser Probiotika besteht in ihrem begrenzten Wirkungsspektrum, weil sie gegen eine kleine Gruppe der Krankheitserreger wirken.

Bekannt ist das Behandlungs- und Prophylaxisbiopräparat Baktisporin für Behandlung eines breiten Kreises der Krankheiten. Das Präparat beinhaltet eine lyophil getrocknete Biomasse des Stamms *Bacillus subtilis* 3H und ein Schutzmedium auf Basis der Saccharose- und Gelatinemischung oder Laktose (Patent RU Nr. 2130316, A61K 35/66, veröffentlicht am 20.05.1999).

Jedoch ist das Biopräparat Baktisporin gegen eine Reihe von Erreger der akuten Darminfektionen nicht wirksam, die z. B. von Bakterien der Gattung Pseudomonas spp., Streptococcus spp., Enterococcus spp. hervorgerufen werden.

Bekannt ist auch das biopharmazeutische «Deprekan -A», das den rekombinanten Stamm *Bacillus subtilis,* der alpha-2-Interferon des Menschen produziert, den Stamm *Bacillus licheniformis* und eine Grundlage beinhaltet. In der Zusammensetzung des Präparats kann der rekombinante Stamm *Bacillus subtilis* ASIM Nr.W-7289 (Allrussische Sammlung der Industriemikroorganismen, im Weiteren ASIM genannt) verwendet werden. Das Präparat verfügt über antibakterielle, antivirale und immunomodulierende Aktivität und kann bei der Behandlung unterschiedlicher Erkrankungen infektiöser und nicht infektiöser Natur verwendet werden. (Patent RU Nr. 2158134, A61K 35/74, veröffentlicht am 20.09.2000).

Jedoch gehört zu der Zusammensetzung des Präparats "Deprekan-A" ein rekombinanter Stamm, der die Resistenz gegen das Antibiotikum (Kanamycin) determiniert. Das wird den Anforderungen von WHO nicht gerecht.

Bekannt ist das Präparat Biosporin zur Vorbeugung und Behandlung der Magen-Darm-Erkrankungen des Menschen. Das Präparat beinhaltet lebende Zellen der Stämme (ASIM Nr.W-2335) und *Bacillus, licheniformis* 31 (ASIM Nr.W-2336) und Grundlage (Patent RU Nr. 1722502, A61 K 35/74, veröffentlicht am 30.03.1992).

Biosporin ist durch eine hohe antagonistische Aktivität gegenüber einem breiten Spektrum der pathogenen und der bedingt pathogenen Mikroorganismen gekennzeichnet (Sorokulowa 1.B., 1997).

Der Mangel von Biosporin besteht darin, dass zu seiner Zusammensetzung gehörende Stämme gegen Antibiotika empfindlich sind. Deshalb wird es nicht empfohlen, das Präparat bei der Behandlung zusammen mit Antibiotika zu verwenden.

Das am nächsten liegende Präparat ist ein Mittel, welches gegen virulente und bakterielle Infektionen bestimmt ist. Das Präparat enthält eine Mischung der Biomasse der Stämme *Bacillus subtilis* ASIM Nr.W-7092, *Bacillus subtilis* ASIM Nr.W-7048, *Bacillus, licheniformis* ASIM Nr.W-7038 in Form der Sporen und mit einem Titer von über 3x10¹⁰ Sporen/g, und Stabilisator - Stärke und Zucker (Patent RU Nr. 2142287, A61 K 35/74, veröffentlicht am 10.12.1999).

Der Mangel des bekannten Präparats besteht darin, dass ein rekombinanter Stamm Plasmide enthält. Die Plasmide determiniert die Resistenz gegen Antibiotika.

In Präparaten der tierärztlichen Anwendung ist der Stamm *Bacillus subtilis* X-15 bekannt. Der Stamm wird für die Vorbeugung und Behandlung von Endometritis bei Kühen (Patent RU Nr. 2180575, C12N1/20, veröffentlicht am 20.03.2002).

Der Mangel des Präparats auf Basis des Stammes *Bacillus subtilis* X-15 besteht darin, dass das Wirkungsspektrum begrenzt wird. Der Stamm ist nur gegen eine kleine Gruppe von Mikroorganismen aktiv, die Endometritis bei Kühen erregen.

Bekannt ist das probiotische Präparat "Subtilis" auf Basis der flüssigen oder liophyl getrockneten Kultur des Stammes *Bacillus subtilis* ASM Nr. W-2250D (Allrussische Sammlung der Mikroorganismen, im Weiteren ASM genannt). Das Präparat ist für die Vorbeugung und Behandlung der Magen-Darm-Erkrankungen der Tiere, des Geflügels und der Fische bestimmt, und sorgt für Erhöhung der Verdaulichkeit der Futtermittel, für Erhöhung der Fruchtbarkeit und Gewichtszunahme (Patent RU Nr. 2184774, C12N1/20, veröffentlicht am 10.07.2002). Zum Mangel des Präparats "Subtilis" gehört ein beschränktes Spektrum der antagonistischen Aktivität.

Das am nächsten liegende Analogon ist ein proboptisches Präparat mit Komplexwirkung. Das Präparat enthält folgende Komponenten, Volumen %: Mischung der Biomasse *Bacillus subtilis* ASIM - 4759 mit Titer 1x10⁷ -10x10⁹ lebender Mikrobenzellen und der Biomasse *Bacillus. Licheniformis* 2336/105 mit Titer 1x10⁸ - 10x10 lebender Mikrobenzellen in 1 ml der physiologischen Lösung- 92-98 %, und Stabilisator auf Basis des Blutserums von Rindvieh, oder der Milch, oder deren Mischung, Saccharose und Gelatine - 2-8 %. Das Präparat hat gleichzeitig die antibakterielle und antivirale Aktivität und ist zu prolongierter Interferonsynthese in unterschiedlichen Teilen der offenen Höhlen des Körpers fähig (Patent RU Nr. 2159625, A61 K 35/66, veröffentlicht am 27.11.2000). Aus der WO2005/019417 A ist die Verwendung einer Ko-Kultur zweier Stämme von Bacillus subtilis und Bacillus licheniformis als probiotischer Nahrungsmittelzusatz bekannt.

Die Aufgabe der Entwicklung besteht in der Gewinnung neuer Stämme *Bacillus subtilis und Bacillus licheniformis* und Entwicklung eines probiotischen Biopräparats auf Basis dieser Stämme. Das Präparat sollte sich durch eine mehr ausgeprägte antagonistische Aktivität gegen bedingt pathogene Mikroorganismen durch eine hohe lysozyme, proteolytische und amylase Aktivität der zum Biopräparat gehörenden Stämme kennzeichnen. Das Präparat sollte gebrauchsfreundlich sein und unterschiedliche Rezepturformen haben.

Das technische Ergebnis der Entwicklung besteht darin, die Effizienz der Vorbeugung und Behandlung der Infektionskrankheiten unterschiedlicher Ätiologie und Dysbiose durch die Verbreitung des Spektrums der antagonistischen Aktivität der zum Biopräparat "Irilis" gehörenden Bakterienstämme zu erhöhen, die Resistenz des Biopräparats gegen Fermente des Magen-Darm-Kanals zu erhöhen, sowie die Gebrauchsfreundlichkeit zu verbessern.

Das Wesen der Entwicklung besteht im Folgenden:
Die zur Zusammensetzung des Biopräparats "Irilis" gehörenden neuen Stämme *B.subtilis* 07 (VKPM B-8611) und *B.licheniformis 09* (VKPM B-8610) werden durch das breite Spektrum der antagonistischen Aktivität, der hohen proteolytischen und amylasen Aktivität *(B.subtilis 07)* und eine ausgeprägte Fähigkeit der Erzeugung von Lysozym *(B. licheniformis 09)* gekennzeichnet. Die Stämme konkurrieren mit einander nicht, sondern nehmen Synergiebeziehungen auf. Das zeigt sich in der Erhöhung der antagonistischen Wirkung des Biopräparats.

Stämme *B.subtilis* 07 (VKPM B-8611.) und *B.licheniformis 09* (VKPM B-8610) sind aus der gesunden Weizenpflanze ausgeschieden, in der Allrussischen Sammlung der Industriemikroorganismen hinterlegt und werden durch folgende Eigenschaften gekennzeichnet:
*Bacillus subtilis* VKPM B - 8611 - grampositive aerobe sporenbildende Stäbchen, 2,7-0,6 x 0,8-0,7 µ groß, die einzeln oder in Form der Ketten liegen. Die Zellen sind bewegbar, bilden unter aeroben Bedingungen ovalenförmige Sporen, die sich in der Zelle zentral anordnen. Bei der Sporenbildung werden die Zellen nicht angeschwollen.

Auf dem Gause-Medium Nr.2, Maische-Agar, auf dem Gromyko-Medium wächst der Stamm reichlich, bildet matte gefaltete fleischfarbige Kolonien mit verschnitteten Kanten, die leicht von Schleifen des Agars abgenommen werden.

Im Protoplasma des Stamms werden die Einschließungen der poly-p-Oxobuttersäure nach dem Wachstum im Glukoseagar nicht aufgefunden. Auf dem Fleischbrühe-Pepton-Nährboden bildet die Kultur ein Häutchen.

Sie wächst unter anaeroben Bedingungen nicht, hydrolisiert den Harnstoff nicht, erzeugt Gas aus Nitraten unter anaeroben Bedingungen nicht, produziert Arginindihydrolase nicht. Die Kultur bildet Katalase. Sie zeigt positive Voges-Proskauer-Reaktion und wächst in Anwesenheit von 7 % NaCl. Sie hydrolisiert Stärke und Kasein, verdünnt Gelatine. Sie fermentiert Glukose, Arabinose, Xylose, Mannit mit Bildung der Säure ohne Gas. Sie reduziert Nitrate, entfärbt das Methylenblau. Sie besitzt die Koagulase- und Lezythinaktivität nicht, besitzt hohe proteolytische Aktivität und Amylase-Aktivität.

Die grundlegenden physikalischen und biochemischen Eigenschaften von *B.subtilis* VKPM B - 8611 sind in den Tabellen 1 und 2 dargestellt.

*Bacillus licheniformis* VKPM B - 8610 - grampositive sporenbildende Stäbchen, 2,6-0,7 x 0,5-06 µ groß. Die Zellen sind beweglich, begeißelt, ordnen sich hauptsächlich in Ketten an. Sporen sind ovalförmig und ordnen sich in der Zelle zentral an. Die Zellen werden bei der Sporenbildung nicht angeschwollen. Nach dem Wachstum im Glukoseagar werden die Einschließungen der poly-p-Oxobuttersäure im Protoplasma nicht aufgefunden.

Auf dem Fleischbrühe-Pepton-Nährboden bildet die Kultur Kolonien mit trüber grober Oberfläche. Die Kolonien sind opak, und sind fest an Agar gebunden: auf der Oberfläche wird oft Schleim gebildet. Auf dem Fleischbrühe-Pepton-Nährboden bildet sich ein gefaltetes Häutchen, manchmal cremefarbenes.

Die Kultur bildet Katalase, kennzeichnet sich durch die Fähigkeit auf dem Agar unter anaeroben Bedingungen zu wachsen. Sie zeigt positive Voges-Proskauer-Reaktion und wächst in Anwesenheit von 7 % NaCl. Sie hydrolisiert Stärke, Kasein, hydrolisiert den Harnstoff nicht. Sie verdünnt Gelatine langsam. Sie fermentiert Glukose, Arabinose, Xylose, Mannit mit Bildung der Säure ohne Gas. Sie reduziert Nitrate, bildet Gas aus Nitraten unter anaeroben Bedingungen. Sie produziert Arginindihydrolase, Lysozym. Sie besitzt die Koagulase- und Lezythinaktivität nicht.

Die grundlegenden Eigenschaften vom Stamm *B.licheniformis* ASIM Nr. W - 8610 sind in den Tabellen 1 und 2 dargestellt.

Die Stämme *B. subtilis* VKPM B - 8611 und *B.licheniformis* VKPM B - 8610 kennzeichnen sich durch eine hohe antagonistische Aktivität gegen ein breites Spektrum der pathogenen und bedingt-pathogenen Mikroorganismen und durch Resistenz gegen eine Reihe von Antibiotika.

In der Zusammensetzung des vorgeschlagenen Biopräparats "Irilis" können die genannten Stämme in unterschiedlichen Kombinationen, z. B. im gleichen Verhältnis (nach dem Titer der Zellen) verwendet werden: Biomasse des Stammes *B. subtilis* VKPM B - 8611 in Titer 1x10⁹ KBE/ml und Biomasse des Stammes *B.licheniformis* VKPM B - 8610 in Titer 1x10⁹ KBE/ml oder in einem Verhältnis in Grenzen (1- 100): (100 - 1), z.B. Biomasse des Stammes *B. subtilis* VKPM B-8611 in Titer 5x10⁹ KBE/ml und Biomasse des Stammes *B. licheniformis* VKPM B - 8610 in Titer 1x10⁹ KBE/ml, oder Biomasse des Stammes *B. subtilis* VKPM B - 8611 in Titer 1x10¹⁰ KBE/ml und Biomasse des Stammes *B.licheniformis* VKPM B - 8610 in Titer 5x10⁹ KBE/ml, oder Biomasse des Stammes *B. subtilis* VKPM B 8611 in Titer 1x10 KBE/ml und Biomasse des Stammes *B.lichenifiormis* VKPM B - 8610 in Titer 1x10⁷ KBE/ml usw.

Das Biopräparat Irilis enthält auch ein Schutzmedium, um die Erhaltung der Bakterien während der technologischen Bearbeitung sicherzustellen, um die Endrezepturform zu erhalten, und nachher aufzubewahren. Als Schutzmedium kann das Präparat z. B. das Saccharose- und Gelatinemedium, die Trockenmilch, Gelatose, Laktose, Saccharose u.ä. beinhalten.

Das Biopräparat "Irilis" kann zusätzlich das Lösungsmittel enthalten. Als Lösungsmittel kann z. B. destilliertes oder abgekochtes Wasser, physiologische Lösung u.ä. verwendet werden.

Das Biopräparat "Irilis" kann zusätzlich eine Grundlage enthalten. Die Grundlage wird gewöhnlich bei der Herstellung unterschiedlicher Rezepturformen verwendet. Bei der Herstellung der Tabletten kann es z. B. Dextrane, Polyglucin, Stärke, Polyvinylpyrrolidon, Saccharose, Laktose, Kalziumstearat, Glukose, Natrium-Hydrogenkarbonat, Aluminiumhydroxid, Methylzellulose, Talk u.a. beinhalten. Bei der Herstellung von Suppositorien oder Zäpfchen enthält es als Grundlage z. B. Süßwarenfett**,** Paraffin, Lanolin, Kakaobutter, Gel des Aluminiumhydroxids u.a.

Das Biopräparat kann auf unterschiedlichen Trägertypen oder Sorbenzien, z.B. auf Aerokraft, Zellulose, A-Kohle, Karboxymethyl-Zellulose, Hydroxyäthyl Zellulose, Chitosan u.a. abgekapselt oder immobilisiert werden.

Das Biopräparat kann auch lyophilisiert werden.

Das Biopräparat "Irilis" kann zur peroralen oder vaginalen Anwendung, oder zur rektalen oder äußeren Anwendung in Form der wässrigen Suspension gebraucht werden. Der Mechanismus der Wirkung des Biopräparats "Irilis" basiert auf der adhäsiven und antagonistischen Aktivität der Bakterien-Probiotika. Die Bakterien-Probiotika produzieren unterschiedliche physiologisch aktive Stoffe und verdrängen pathogene und bedingt pathogene Mikroorganismen aus dem Verdauungstrakt des Makroorganismus. Sie wirken stimulierend auf spezifische und nicht spezifische Schutzreaktionen des Makroorganismus.

### Die besten Varianten der Ausführung der Erfindung

Die Entwicklung wird anhand folgender Beispiele veranschaulicht.

**Beispiel 1**. Herstellung des flüssigen Präparats.

Stämme werden einzeln oder auf festen oder auf flüssigen Nährboden kultiviert.

Bei der stationären Technologie werden die Stämme auf den festen Agrarnährböden in Matratzen oder Glasflaschen in Thermoschwengelmaschine unter Temperatur ab 22 bis 38° C innerhalb von ab 12-16 std. bis zu 7 Tage kultiviert. Nach der Beendigung der Inkubation wird die Biomasse, die auf dem Nährboden gewachsen ist, durch den Stabilisator- Schutzmedium, das die 5 %-Laktoselösung enthält, im Verhältnis 10:1 zusammengebracht und in Flaschen abgezogen. Es wird das Biopräparat mit dem Gehalt der Biomasse des Stammes *B. subtilis* VKPM B - 8611 in Titer 1x1.0¹⁰ KBE/ml und Biomasse des Stammes *B.licheniformis* VKPM B *-* 8610 - 1x10⁹ KBF/ml.

Bei der industriellen Technologie werden die Stämme in einem Reaktor/Fermenter mit einem Nährboden für Kultivierung unter Temperatur 35- 38° C innerhalb von 10-18 Stunden kultiviert. Der Prozess gilt als abgeschlossen, wenn die Konzentration der Zellen 4-5 Mlrd/ml und das Verhältnis der Sporen und vegetativen Zellen 1:1 beträgt. Nach der Beendigung der Inkubation werden separat gezüchtete Kulturen im Verhältnis 1:1 zusammengebracht, das Saccharose-Gelatine-Schutzmedium zugesetzt und in Flaschen abgezogen. Es wird das Biopräparat mit dem Gehalt der Biomasse des Stammes *B. subtilis* VKPM B - 8611 in Titer 5x10⁹ KBE/ml und Biomasse des Stammes *B.licheniformis* VKPM B - 8610 - 5x10⁹ KBE/ml.

**Beispiel 2.** Herstellung des Präparats in Form von Lyophilisat.

Bei der stationären Technologie werden die Stämme auf den festen Agarnährböden in Matratzen oder Glasflaschen in Thermoschwengelmaschine unter Temperatur ab 22 bis 38° C innerhalb von ab 12-16 Std. bis 7 Tage kultiviert. Nach der Beendigung der Inkubation wird die Biomasse, die auf dem Nährboden gewachsen ist, durch den Stabilisator- Schutzmedium, das die 10 %-Glyzerinlösung enthält, abgewaschen, in Ampullen(Flaschen) oder in Edelstahlkassetten abgezogen. Danach wird sie eingefroren und in der Vakuum-Trocknungs-Anlage entwässert oder mittels eines Sprühverfahrens getrocknet.

Bei der industriellen Technologie werden die Stämme in einem Reaktor/Fermenter mit einem Nährboden für Kultivierung unter Temperatur 35-38° C innerhalb von 10-18 Stunden kultiviert. Der Prozess gilt als abgeschlossen, wenn die Konzentration der Zellen 4-5 Mlrd/ml und das Verhältnis der Sporen und vegetativen Zellen 1:1 beträgt. Nach der Beendigung der Inkubation werden separat gezüchtete Kulturen im Verhältnis 2:1 zusammengebracht, in den Stabilisator zugegeben, in Ampullen (Flaschen) oder in Edelstahlkassetten abgezogen. Danach wird sie eingefroren und in der Vakuum-Trocknungs-Anlage entwässert oder mittels eines Sprühverfahrens getrocknet.

**Beispiel 3.** Herstellung des Präparats in Form von Tabletten.

Gezüchtete Kulturen der Stämme *B. subtilis* VKPM B - 8611 und *B.licheniformis* VKPM B - 8610 werden nach der Zugabe der Komponenten des Aufschwemmungsmediums an der Vakuum-Troknungs- oder Sprüh-Troknungs-Anlage entwässert, mit Zuckergranulat und Gleitstoffen (Stärke oder Kalziumstearat) zusammengebracht und an der Rotationspressen gepresst.

Es wurde 10 experimentellen Industrieserien des Präparats in Form von Tabletten auf Stabilität geprüft. Gemäß ermittelten Ergebnissen beträgt der Gehalt der lebenden *B. subtilis* VKPM B -8611 und *B.licheniformis* VKPM B - 8610 in Tabletten unmittelbar nach der Pressung über 10⁹⁻⁷ bzw. 10 ⁸⁻⁶ KBE/Dosis. Nach der Aufbewahrung der Tabletten innerhalb von 12 Monaten wurde der Gehalt der Mikrobenzellen in keiner Partie kleiner geworden! O⁹⁻⁷-10⁸⁻⁶ KBE/Dosis.

**Beispiel 4.** Herstellung des Präparats in Form von Suppositorien.

Gezüchtete Kulturen der Stämme *B. subtilis* VKPM B - 8611 und *B.licheniformis* VKPM B - 8610 werden nach der Zugabe der Komponenten des Aufschwemmungsmediums an der Vakuum-Trocknungs- oder Sprüh-Trocknungs-Anlage entwässert, mit Grundlagen (Süßwarenfett oder Paraffin) zusammengebracht und an einer speziellen Anlage Suppositorien gegossen.

Es wurde 10 experimentellen Industrieserien des Präparats auf Stabilität geprüft. Gemäß ermittelten Ergebnissen beträgt der Gehalt der lebenden *B. subtilis* VKPM B 8611 und *B.licheniformis* VKPM B - 8610 in Suppositorien unmittelbar nach der Pressung über 10⁹⁻⁷ bzw. -10 ⁸⁻⁶ KBF/Suppositorium. Nach der Aufbewahrung der Suppositorien innerhalb von 12 Monaten wurde der Gehalt der lebenden Mikrobenzellen in keiner Partie unter 10⁹⁻⁷ - 10⁸⁻⁶ KBE/Suppositorium reduziert

**Beispiel 5.** Alle gemäß Beispielen 1, oder 2, oder 3, oder 4 hergestellte Varianten und Formen des Biopräparats "Irilis" werden auf die Unschädlichkeit an Laboratorien, auf spezifische antagonistische Aktivität gegen Testkulturen-Vertreter unterschiedlicher Gruppen von pathogenen und bedingt pathogenen Mikroorganismen, auf die Resistenz gegen Antibiotika geprüft.

Das Biopräparat "Irilis" kennzeichnet sich durch die Unschädlichkeit.

Für die Feststellung der Unschädlichkeit wird der Inhalt der Flasche in 0,5 ml der physiologischen Lösung verdünnt. Diese Dosis wird peroral den Mäusen eingeführt.

Für jede Versuchsvariante werden über 10 Mäuse mit der Masse 15-16 g gebraucht. Das Präparat gilt als unschädlich, wenn alle Mäuse innerhalb von 5 Tage der Bobachtung lebendig sind und bei keiner Maus eine Erkrankung festgestellt wird.

Das Biopräparat "Irilis" kennzeichnet sich durch ein breites Spektrum der antagonistischen Aktivität gegen Teststämme der Kulturen der pathogenen und bedingt pathogenen Mikroorganismen. In der Tabelle 3 ist die Charakteristik der antagonistischen Aktivität des Präparats gegen pathogene und bedingt pathogene Mikroorganismen dargestellt, die bei unterschiedlichen Erkrankungen und Dysbiose unterschiedlicher Ätiologie ausgeschieden sind. Für die Ermittlung der spezifischen Aktivität wird die antagonistische Aktivität der Varianten des Präparats gegen klinische Testkulturen untersucht. Die Untersuchung wird mittels des Verfahrens des verzögerten Antagonismus durchgeführt. Dafür wird der Inhalt der Flasche in 1 ml der physiologischen Lösung verdünnt. Die erhaltene Suspension wird mit Strichen auf den Durchmesser der Petrischale mit Gause-Agarnährboden Nr. 2 gesät. Die Einsäen werden im Thermostat unter Temperatur 37° C innerhalb von 72 Stunden inkubiert. Dann werden zur gewachsenen Kultur mit Strichen die Testorganismen (500 - Millionen-Suspensionen der Tageskulturen in einer physiologischen Lösung) nachgesät. Die Auswertung der Ergebnisse erfolgt nach 18 Stunden der Inkubation unter 37° C nach der Größe der Zonen, wo die Testkulturen nicht gewachsen sind.

Als Kontrolle des Wachstums der Testkulturen dient deren parallele Züchtung in Schalen mit Gause-Agarmedium Nr.2 ohne untersuchte Kultur.

Aus Angaben der Tabelle 3 folgt es, dass optimale Anzahl der lebenden Zellen in einer Dosis des Präparats 1 - 5x10⁹ ist. Eine weitere Erhöhung der Anzahl der Mikrobenzellen ändert die antagonistische Aktivität des Präparats gegen Testkulturen der Mikroorganismen wesentlich nicht.

**Beispiel 6.** Es wird die Behandlungseffizienz des Probiotikums nach dem Beispiel 1 oder 2 oder 3 im Vergleich zum Präparat, Pektosorbin" in einem Betrieb ermittelt. Im Betrieb haben Ferkel Magen-Darm-Erkrankungen mit Durchfallsymptomen. Es werden 3 Gruppen aus 81 Ferkel, die 1-2 Tage alt sind, mit klinischen Merkmalen von Dyspepsie (Durchfall, dünnflüssiger Stuhl, Kot hat einen saueren Geruch, Kot hat Gasblasen, Appetitlosigkeit, Trockenheit und Blässe der sichtbaren Schleimhäute, Pulsbeschleunigung, gedämpfter Herzton, beschleunigte Atmung) gebildet.

Nach einer klinischen Untersuchung wurde es festgestellt, dass 69 Ferkel eine einfache Dyspepsie, und 12- eine toxische Dyspepsie haben.

Bei den bakteriologischen Untersuchungen der Kotproben werden pathogene Kolibazillen in großen Mengen (10⁸ -10¹¹ Bakterien in 1 g) ausgesondert, wenn die Stämme der Kolibazillen- der normalen Vertreter des Dickdarms nur bei 8 Ferkel (10³ -10⁴ Bakterien in 1 g) ausgesondert werden.

Pathogene Kolibazillen, im Unterschied zu den normalen Vertretern des Darms fermentieren in der Regel keine Laktose, bringen weiße Mäuse nach der intraabdominalen Einführung um. Nach der Serotypisierung der von Ferkeln ausgelaugten Stämme wurden 11 Stämme zur enteropathogenen Serogruppe 0141 gezählt, die restlichen 40 Stämme wurden durch typenspezifische Seren nicht agglutiniert. Die Ergebnisse der Untersuchungen zeugen über die Mikroflorastörung des Dickdarms der an Dyspepsie leidenden Ferkel (Dysbiose). Das ist der Grund deren Erkrankungen.

Die Ferkel der ersten und zweiten Gruppen bekommen 2, bzw. 4 Dosen des Präparats "Irilis" 2mal am Tag mit einer Intervalle 12 Stunden erst nach 3-4 stündigen Fasten und Wasserdiät 20-30 Minuten vor dem Füttern. Das Präparat wird bis zur kompletten Genesung gegeben. Die Ferkel der dritten Gruppe bekommen das Präparat "Pektosorbin" in Dosen 0,26 g für 1 kg der Masse mit Kolostrum laut Gebrauchsanweisung dreimal am Tag bis zur kompletten Genesung. Die Ferkel werden klinisch beobachtet. Es wird die Anzahl der genesenden Tiere, die Dauer der Behandlung berücksichtigt. Die Genesung wird beurteilt, wenn die Merkmale des Durchfalls, die pathologische Kotbeimischungen, Blähung, Krämpfe verschwinden, der Allgemeinzustand verbessert wird, der Appetit erscheint. Die Ergebnisse sind in der Tabelle 4 zusammengeführt.

Wie es in Tabelle 4 ersichtlich ist, sichert das Probiotikum hohe Effizienz bei der Anwendung 4 Dosen zweimal am Tag. Die Genesung kommt nach 4-6maligen Anwendung von "Irilis", wenn Diätregime eingehalten wird. Die Normalisierung der Darmmikroflora beseitigt Dehydratation. Das schließt die Notwendigkeit aus, die Maßnahmen zur Rehydration einzuleiten. Nach dem Aufhören der Präparatseinnahme wird die Erkrankung nicht registriert.

**Beispiel 7.** Es wird die Effizienz des Präparats "Irilis" bei der Behandlung der Kälber mit Durchfallssymptome ermittelt und die vorbeugende Wirkung auf Tiere bis zum zweiten Lebensmonat eingewertet. Dafür wurden 3 Gruppen von gesunden neugeborenen Kälbern (1-2 Tage) mit dem Gewicht bei der Geburt 28-30 kg gebildet. Kälber aller Gruppen werden gemäß den Empfehlungen über Zuchttechnologie gefüttert: 1,5-2 I des Kolostrums (Milch) pro jede Fütterung 3mal am Tag. Zum ersten Mal wird das Kolostrum spätestens in 1,5 stunden nach der Geburt gegeben. Die Kälber von ersten zwei Gruppen (40 Stück in der Gruppe) bekommen 20-30 Min vor der zweiten Fütterung innerhalb der ersten fünf Tage in 2 Kursen mit einer Pause von 3-4 Tage zwischen den Kursen das Präparat "Irilis" je 1, bzw. 2 Dosen. Vor der Einführung des Präparats wird der Inhalt einer Ampulle (Flasche) in 100 ml des abgekochten und abgekühlten bis zur Temperatur 38-39° C Wassers gelöst. Die Kälber der ersten Versuchsgruppe bekommen 50 ml, die Kälber der zweiten Versuchsgruppe - 100 ml des Präparats. Dritte Gruppe - Kontrollgruppe (20 Kälber) bekommt das Präparat "Irilis" nicht.

Kälber werden nach der Geburt und in 2 Monaten gewogen. Täglich wird der Körperzustand, Appetit, Funktion des Verdauungssystems (Art des Stuhls, Blähung), Verhalten des Tieres berücksichtigt. Am Ende des Versuchs werden Blutproben der 5 Kälber aus jeder Gruppe für hämatologische Untersuchungen entnommen. Im Blut wirde der Hämoglobingehalt mit Hilfe des Sahli-Hämometers, Gehalt der Blutzellen in Gorjaew-Zählkammer, Leukozytenformel mit Hilfe des Verfahrens, Gesamteiweißkonzentration im Blutserum refraktometrisch, Gehalt der Immunoglobuline mit Hilfe des Zinksulfat-Tests, Gehalt von Lysozym nach E.W.Ermolajewa-Methode, bakterizide Aktivität nach S.W.Smirnowa-T.A.Kusmina-Methode gestgestellt. Die Ergebnisse der Untersuchungen sind in der Tabelle 5 dargestellt.

Wie es in Tabelle 5 ersichtlich ist, beeinflusst die Anwendung des Probitikums "Irilis" auf die Hämoglobinkonzentration im Blut, Anzahl der Blutzellen und Leukozytenformel wesentlich nicht. Des Weiteren steigt der Spiegel des Gesamteiweißes im Blutserum, der Immunoglobuline, des Lysozyms, und der bakteriziden Aktivität des Blutserums bei den Kälbern, die das Präparat bekommen haben.

Es wurde festgestellt, dass "Irilis" keinen negativen Einfluss auf die Gesundheit der Tiere nimmt. Es erhöht deren Resistenz gegen Magen-Darm-Erkrankungen, verbessert die Lebensqualität der Kälber. Kälber verlieren das Gewicht nicht. (Tabelle 6).

Wie es in Tabelle 6 ersichtlich ist, sind in Kontrollgruppe, die kein "Irilis" bekommen hat, 15 von 20 Kälbern krank geworden, was 75 % der Tiere in der Gruppe beträgt. In erster Versuchsgruppe (1 Dosis von "Irilis" - 1mal am Tag) sind 11 von 40 Kälbern (27,5 %), in zweiter Versuchsgruppe (2 Dosen von "Irilis" - 1mal am Tag) - 7 von 40 Kälbern (17,5 %) krank geworden. Die Vorbeugungseffizienz des Probitikums "Irilis" beträgt 72,5 %, bzw. 82,5 %. Dabei werden die Kälber der Kontrollgruppe für 2 und 9 Tage krank, und die Krankheit verläuft hauptsächlich in schwerer Form. Im Ergebnis beträgt der Abgang 30 %, und Erhaltung - 70 %. Die Anwendung des Präparats "Irilis" in Menge über 2 Dosen ist unzweckmäßig. Das wird durch hohe Prozentzahlen der Erhaltung der Kälber und tagesdurchschnittliche Gewichtszunahme bestätigt, 95 %, bzw. 9 %.

**Beispiel 8.** Es wird die Anwendungseffizienz des Präparats "Irilis" zur Vorbeugung der Magen-Darm-Erkrankungen mit Durchfallsymptomen im Vergleich zu den Präparaten "Pektosorbin" und Immunoglobulin in einem Problembetrieb ermittelt. Es werden 3 Gruppen je 35 Kälber von 105 neugeborenen Kälbern gebildet. Alle Tiere sind gesund, normal entwickelt und der Unterschied zwischen den Gruppen nach dem durchschnittlichen Gewicht beträgt bis zu 2 %.

Kälber aus der ersten Gruppe bekommen das Präparat "Irilis" (2 Dosen) 20-30 vor zweiter Fütterung mit Kolostrum. Kälber aus zweiter Gruppe bekommen "Pektosorbin" gemäß der Gebrauchsanweisung. Kälber aus dritter Gruppe bekommen nicht spezifisches Immunoglobulin intramuskulär gemäß der Gebrauchsanweisung.

Die Vorbeugungskurse in Gruppen dauern: In erster Gruppe -10 Tage, in zweiter Gruppe - 10 Tage, in dritter Gruppe - 50 Tage. Beobachtungszeitraum beträgt 60 Tage. Kälber werden nach der Geburt, in einem Monat und am Ende des Beobachtungszeitraums gewogen. Täglich wird der klinische Zustand beobachtet, es wird auf die Anzahl der kranken Tiere, auf den Verlauf und Krankheitsausgang geachtet. Die Ergebnisse sind in der Tabelle 7 dargestellt.

Wie es in Tabelle 7 ersichtlich ist, ist die Effizienz des Probiotikums "Irilis" im Vergleich zu den bekannten Präparaten Pektosorbin und Immunogobulin wesentlich höher. Das wird durch die Werte der Erhaltung und tagesdurchschnittlichen Gewichtszunahme bestätigt.

**Beispiel 9.** Es wird die Möglichkeit der Einführung des probiotischen Präparats ins traditionelle Fütterungsschema in Geflügelzucht bei der Broilermast untersucht. Es werden 4 Gruppen Hühnerküken gebildet. Die bekommen:
1. Gruppe - Präparat «Wetom», hergestellt von NPF-IZ «Koltsowe»,
2. Gruppe - Präparat "Irilis"
3. Gruppe - Präparat STF, hergestellt von OOO «Bioawtomatika»,
4. Gruppe - Präparat «Enterosporin»

Das experimentale Vorbeugungsschema nach Zuchttagen sieht folgenderweise aus:
1. Tag - Probiotika mit Futtermittel
1-3. Tage - Flourchinolon
4-13. Tage - Probiotika mit Futtermittel

Im Weiteren gemäß dem in Betrieben verwendeten Vorbeugungsschemas.

Die Auswertung der Ergebnisse erfolgt gemäß grundlegenden technologischen Werten: Gewicht der Broiler am 1., 7., 14., 28., 35., und 42. Tag und beim Schlachten; Sterberate jede Woche und Gesamtsterberate vor dem Schlachten. Die Ergebnisse sind in der Tabelle 8 dargestellt.

Die in der Tabelle 8 angeführten Angaben zeugen darüber, dass die Anwendung des Präparats "Irilis" in der Broilerzucht ermöglicht es, ein hohes Ergebnis der Bestandserhaltung zu bekommen. Das Präparat "Irilis" wirkt positiv auf den Broilerorganismus und sorgt für eine größere Gewichtszunahme im Vergleich zur Kontrolle.

So haben die durchgeführten Untersuchungen des Präparats "Irilis" gezeigt, dass seine Anwendung möglich und hoch effektiv bei der Vieh- und Geflügelzucht ist. Es stellt Inhibition der pathogenen Kolibazillen, Beibehaltung einer optimalen Mikrobenbilanz im Verdauungstrakt, Erhöhung der nicht spezifischen Resistenz der Tiere, deren Erhaltung und Gewichtszunahme sicher. Es hat einen Vorbeugungs- und Behandlungseffekt bei den Erkrankungen, die mit dem Durchfall begleitet werden, und vorbeugt die Dysbiose. Der Vorteil des Präparats "Irilis" besteht in der Chromosomenresistenz der enthaltenen Stämme gegen Antibiotika. Das ermöglicht seine Anwendung im Komplex der mikrobiziden Therapie.

**Tabelle 1**

| | | |
|---|---|---|
| Kultureigenschaften, morphologische und biochemische Eigenschaften | | |

| Eigenschaften | *B. subtilis VKPMB-8611* | *B. licheniformis VKPMB-8610* |
|---|---|---|
| Wachstum unter anaeroben Bedingungen | - | + |
| Fermentation | | |
| - Glukose | + | + |
| - Arabinosen | + | - |
| - Xylosen | + | + |
| - Mannite | + | + |
| Verwertung | + | + |
| - Zitrat | | |
| - Propionat | - | + |
| Hydrolyse | + | + |
| - Stärke | | |
| - Harnstoff | - | - |
| - Kasein | + | + |
| - Thyrosin | - | - |
| Reduktion der Nitrate | + | + |
| Bildung des Gases aus NO₃ unter anaeroben Bedin-gingen | - | + |
| Entfärben des Methylenblaus | + | + |
| Arginindihydrolase | - | + |
| Lezithinase | - | - |
| Hyaluronidase | - | - |
| Hämolytische Aktivität | - | - |
| Bildung der Globuli der poly-p-Oxybuttersäure im Glukoseagar | - | - |
| Lysozymaktivität | + | + |
| Wachstum unter 50 °C | + | + |
| Wachstum in 7% NaCl | + | + |
| Wachstum unter 65 °C | - | - |
| Säure- und Gasbildung aus Glukose | - | - |
| Säurebildung aus Glukose | + | + |

**Tabelle 2**

| | | |
|---|---|---|
| Antibiotika-Empfindlichkeit der Stämme der Gattung *Bacillus* | | |

| Untersuchtes Präparat | Durchmesser der Zonen der Verzögerung des Kultur-wachtums, mm | |
|---|---|---|
| | *B. subtilis VKPM B-8611* | *B.licheniformis VKPM B-8610* |
| PENIZILLINE | | |
| Aslozillin | 19±0,1 | 16+0,1 |
| Amoxyzillin | 19±0,2 | 16+0,1 |
| Ampizillin | 11±0,2 | 0 |
| Carbenizillin | 23±0,5 | 20+0,1 |
| Mezlozillin | 21±0,2 | 15+0,2 |
| Methicillin | 20±0,1 | 10±0D |
| Oxazillin | 15±0,3 | 11+0,1 |
| Benzylpenizillin | 7±0,1 | 3±0,2 |
| Piperazillin | 15+0,3 | 11+0,1 |
| Ticarzillin | 25±0,4 | 21+0,2 |
| CARBAPENEME | | |
| Imipenem | 38±0,2 | 30+0,1 |
| ZEPHALSPORINE | | |
| Moxalactam | I±0.2 | 12+03 |
| Cefalotin | 35±0,1 | 21 +0,3 |
| Cefasolin | 25±0,2 | 19+0,2 |
| Cefamandol | 35±0,3 | 15+0,1 |
| Cefoxitin | 18±0,1 | 13+0,1 |
| Cefoperazon | 15±0,2 | 13+0,1 |
| Cefotaxim | 15±0,1 | 9+0,1 |
| Zeftazidium | 6±0,3 | 13+02 |
| Ceftizoxim | 3±0,1 | 11±0,1 |
| Ceftriaxon | 20+0,4 | 12+0,2 |
| Cefuroxim | 2±0,1 | 3±0,1 |
| AMINOGLYKOSIDE | | |
| Amikacin | 22±0,3 | 17+0,2 |
| Gentamicin | 25±0,3 | 19+0,2 |
| Kanamicin | 22±0,2 | 21+0,1 |
| Tobramicin | 25±0,1 | 20+0,2 |
| ANDERE | | |
| Vancomicin | 12±0,1 | 10±0,1 |
| Klindamicin | 9+0,2 | 12+0,1 |
| Tetrazyklin | 26+0.1 | 24+0,1 |
| Chloramfenikol | 18±0,3 | 10±0,1 |
| Polymixin E | 11+0,1 | 10+0,2 |
| Nitrofurantoin | 15+0,2 | 20+0,1 |
| Trimethoprim | 23±0,1 | 25+0,2 |
| Bactrim | 29±0,2 | 31+0,3 |
| Norfloxacin | 24±0,2 | 25+0,2 |
| Nalidixinsäure | 21±0,1 | 15+0,1 |

**Tabelle 3**

| | | | | | |
|---|---|---|---|---|---|
| Antagonistische Aktivität der Variante des Biopräparats "Irilis" gegen klinische Stäm-me der pathogenen und bedingt pathogenen Mikroorganismen | | | | | |

| Testkulturen | Varianten des Biopräparats (Verhältnis B.subtilis: B.licheniformis) x 10⁸"⁶ KBE/ml Zonen der Verzögerung des Wachstums der Test-stämme (mm) | | | | |
|---|---|---|---|---|---|
| | 1:1 | 2:1 | 5:1 | 1:10 | 10:1 |
| Shigella sonne (n=25) (von Ruhrkranken) | 10-12 | 12-15 | 12-15 | 12-15 | 12-15 |
| Salmonella typhimurium (n=25) (von Sal-monellose-Kranken) | 10-13 | 12-18 | 12-18 | 12-18 | 12-18 |
| Escherihia coli 0:157:H7 (n=13) (von Kranken an enterohamorrhagischen Koli-tis und von Tieren) | 9-10 | 11-13 | 11-13 | 11-13 | 11-13 |
| Staphylococcus aureus (n=29) (bei Darmdydysbiore ) | 10-15 | 15-20 | 15-20 | 15-20 | 18-20 |
| Staphylococcus aureus (n=45) (bei Eiter- und Entzündungserkrankungen) | 12-16 | 15-18 | 15-20 | 18-20 | 22-25 |
| Staphylococcus aureus (n=20) (bei Schei-dedysbiore) | 15-18 | 15-20 | 18-22 | 18-22 | 20-25 |
| Proteus vulgaris (n=18) (bei Darmdysbiore) | 10-15 | 15-20 | 15-20 | 15-20 | 15-20 |
| Proteus vulgaris (n=18) (von Pyelonephritiskranken) | 10-12 | 12-15 | 12-15 | 12-15 | 12-15 |
| Proteus mirabilis (n=15) (bei Darmdysbiore) | 15-18 | 18-20 | 18-20 | 18-20 | 18-20 |
| Candida albicans (n=42) (bei Darmdysbiore) | 25-30 | 25-30 | 25-30 | 25-30 | 25-30 |
| Candida albicans (n=20) (bei Scheiddysbiore) | 25-30 | 25-30 | 25-30 | 25-30 | 25-30 |
| Escherihia coli (n=18) (von Pyelonephritiskranken) | 20-22 | 22-25 | 22-25 | 22-25 | 22-25 |
| Escherihia coli (n=15) (bei Eiter- und Entzündungserkrankungen) | 10-12 | 12-15 | 12-15 | 12-15 | 12-15 |
| Streptococcus (n=17) (bei Eiter- und Entzündungserkrankungen) | 10-12 | 12-15 | 12-15 | 12-15 | 12-15 |

**Tabelle 4**

| | | | |
|---|---|---|---|
| Vergleichseffizienz der Anwendung von "Irilis" und "Pektosorbin" für die Behand-lung der Magen-Darm-Erkrankungen der Ferkel, die von der pathogenen Mikro-flora erregt sind | | | |

| Merkmale | Gruppe der Ferkel | | |
|---|---|---|---|
| | erste, n=27 Irilis 2 Dosen | zweite, n=27 Irilis 4 Dosen | dritte, n=27 Pektosorbin |
| Genesen, Stück | 24 | 26 | 20 |
| Gefallen oder notgedrungen vernichtet, Stück | 3 | 1 | 7 |
| Behandlungskurs, Tage | 4,5±0,4 | 2,0±0,6 | 6,5±0,6 |
| Behandlungseffizienz, % | 88,9 | 96,3 | 74,1 |

**Tabelle 5**

| | | | |
|---|---|---|---|
| Untersuchung der grundlegenden Bluteigenschaften der 2-Monat-Kälber nach den Vorbeugungsmaßnahmen | | | |

| Merkmale | Gruppen der Kälber | | |
|---|---|---|---|
| | Versuchsgruppe 1, (n=40) | Versuchs-gruppe 2, (n=40) | Kontrollgruppe 3, (n=20) |
| Hämoglobin, g/l | 127,0±13,6 | 132,7+4,0 | 126,0+3,0 |
| Erythrozyten, | 7,2±0,2 | 7,4±0,4 | 7,2±0,2 |
| Leukozyte, | 6,4±1,0 | 6,3±0,9 | 6,8±0,7 |
| Gesamteiweiß, g/l | 62,1±1,6 | 70,5±1,7 | 61,0±2,6 |
| Gesamtimmunoglobuline, g/l | 29,4±1,9 | 38,0±2,1 | 27,4±1,7 |
| Lysozym, mkg/l | 770,0±31,0 | 980,0±22,0 | 765,0+33,0 |
| Bakterizide Aktivität, % | 67,0±0,5 | 86,5±2,0 | 60,2±1,7 |
| Leukozytenformel, %: | | | |
| Basophile | - | - | - |
| Eosinophile | 4 | 3 | 3 |
| Neutrophile: | | | |
| -jung | - | - | - |
| - stäbchenkernig | 3 | 3 | 2 |
| - segmentkernig | 25 | 23 | 24 |
| Lymphozyte | 66 | 68 | 69 |
| Monozyte | 2 | 3 | 2 |

**Tabelle 6**

| | | | |
|---|---|---|---|
| Vorbeugungseffizenz von Irilis bei der Zucht der Milchkälber | | | |

| Merkmale | Gruppen der Kälber | | |
|---|---|---|---|
| | Erste, n=40 | Zweite, n=40 | Dritte, n=20 |
| Erkrankt, Stück (%) | 11(27,5) | 7(17,5) | 15(75,0) |
| Im Alter, Tage | 3,5±0,3 | 3,5±0,1 | 1,5±0,2(6 St.) 8,5±0,8 (9 St.) |
| Verlauf der Krankheit, St. (%) | | | |
| - leicht | 4(10,0) | 5(12,5) | 5(25,0) |
| - schwer | 7(17,5) | 2(5,0) | 10(50,0) |
| Gefallen im Beobachtungszeitraum, Stück (%) | 2(5,0) | | 4(20,0) |
| Notgedrungen vernichtet, Stück (%) | 6(20,0) | 2(5,0) | 2(10,0) |
| Erhaltung, Stück (%) | 32(80,0) | 38 (95,0) | 14(70,0) |
| Tagesdurchschnittliche Gewichtszu-nahme, g(%) | 665,0±39,0 (104,0) | 700,0+27,0(109,4) | 640,0±25,0 (100,0) |

**Tabelle 7**

| | | | |
|---|---|---|---|
| Vergleichseffizienz der Anwendung von "Irilis" und bekannten Präparaten für die Vorbeugung der Magen-Darm-Erkrankungen, die von "Stall"-Mikroflora erregt wer-den, bei neugeborenen Kälbern | | | |

| Merkmale | Gruppen der Kälber | | |
|---|---|---|---|
| | Erste, n=35 | Zweite, n=35 | Dritte, n=35 |
| | Art der Vorbeugung | | |
| | Irilis | Pektosorbin | Immunoglobulin |
| Erkrankte Kälber, Stück | 2 | 8 | 7 |
| Davon gefallen, St. | - | 5 | 3 |
| Diagnose | Dyspepsie | Toxische Dyspepsie | Toxische Dyspepsie |
| Verlauf der Krankheit, St.: | | | |
| - schwer | - | 5 | 4 |
| - leicht | 2 | 3 | 3 |
| Vorbeugungsseffizienz, % | 94,3 | 77,1 | 80,0 |
| Erhaltung, % | 100,0 | 80,0 | 91,4 |
| Tagesdurchschnittliche Ge-wichtszunahme, g | 820,0 | 680,0 | 660,0 |

**Tabelle 8**

| | | | | | | | | | | | | | | |
|---|---|---|---|---|---|---|---|---|---|---|---|---|---|---|
| Ergebnisse der Untersuchung der Präparate-Probiotika auf Broiler | | | | | | | | | | | | | | |

| Gruppe | Nº der Halle | Anzahl, Stück | PRÄPARAT | | | ERGEBNISSE | | | | | | | | |
|---|---|---|---|---|---|---|---|---|---|---|---|---|---|---|
| | | | Bezeichnung | Dosis | Abgabe-frist, Tage | Zuchtdauer | | | | | | | Bei Abgabe zur Schlachtung (46 T) | |
| | | | | | | 1 Tag | 7 T | 14 T | 21 T | 28 T | 35 T | 42 T | durch-schnittliche Lebend-tagzunah-me, g | Sterbe, Gesamt-rate, % |
| NORM DES GEWICHTS, g/St. | | | | | | | 120 | 310 | 590 | 950 | 1370 | 1805 | 42,0 | |
| 1. Ver-such | 46 | 30371 | WETOM | 75mg/kg des Ge-wichts | 1).1. 2) 4.-13. | GEWICHT % gegenüber Norm | | | | | | | 37,9 | 4,19 |
| | | | | | | 39g | 74,2 | 74,2 | 88,9 | 84,8 | 91,6 | 90,5 | | |
| | | | | | | STERBENRATE % gegenüber Norm | | | | | | | | |
| | | | | | | 0,4 | 0,4 | 0,5 | | 0,56 | 1,32 | 0,8 | | |
| 2. Ver-such | 42 | 30660 | IRILIS | 4mg/St. | ->- | GEWICHT % gegenüber Norm | | | | | | | 37,6 | 3,96 |
| | | | | | | 39g | 81,7 | 77.1 | 80,8 | 82.9 | 86,0 | 87,0 | | |
| | | | | | | STERBENRATE % gegenüber | | | | | | | | |
| | | | | | | 0,82 | | 0,65 | 0,46 | 0,47 | 0,65 | 0,66 | | |
| 3. Ver-such | 18-2 | 19642 | STF | 0,5 mg/St. | ->- | GEWICHT % gegenüber Norm | | | | | | | 39,9 | 4,97 |
| | | | | | | 39g | 79,2 | 80,0 | 85,4 | 85,8 | 92,6 | 91,9 | | |
| | | | | | | STERBERATE % gegenüber | | | | | | | | |
| | | | | | | 0,79 | 0,67 | 0,64 | | 0,8 | 0,8 | 0,76 | | |
| 4. Kon-trolle | 50 | 30575 | ENTEROSPORIN | 100 ml/5 Taus. St. | 4. und 5. | GEWICHT % gegenober Norm | | | | | | | 37,2 | 3,91 |
| | | | | | | 39g | 80,0 | 78,1 | 76,8 | 81,0 | 84,9 | 83,7 | | |
| | | | | | | STERBENRATE % gegenüber | | | | | | | | |
| | | | | | | 0,45 | 0,7 | 0,54 | | 0,46 | 0,66 | 0,7 | | |

## Patentansprüche

1. Biopräparat zur medizinischen und tierärztlichen Anwendung zur Vorbeugung und Behandlung von Infektionserkrankungen und Dysbiose unterschiedlicher Ätiologie, das lebende Mikrobenmasse der Stämme Bacillus subtilis, Bacillus licheniforms und Schutzmedium enthält,
**dadurch gekennzeichnet,**
**dass** es von Stämmen *Bacillus subtilis* den Stamm *Bacillus subtilis* VKPM B - 8611, und von Stämmen *Bacillus licheniformis* den Stamm *Bacillus licheniformis* VKPM B - 8610 in effektiver Menge enthält.

2. Biopräparat nach Anspruch 1,
**dadurch gekennzeichnet,**
**dass** es zusätzlich eine Grundlage enthält.

3. Biopräparat nach Anspruch 2,
**dadurch gekennzeichnet,**
**dass** es als Grundlage Protein, organisches Polymer, Milch, Serum, Albumin, Saccharose, Laktose, Kalziumstearat, Glukose, Natrium-Hydrogenkarbonat, Aluminiumhydroxid, Methylzellulose, Talk Süßwarenfett, Paraffin, Lanolin, Kakaobutter, Gel des Aluminiumhydroxids beinhaltet.

4. Biopräparat nach Anspruch 3,
**dadurch gekennzeichnet,**
**dass** es von organischen Polymeren Dextrane, Polyglycin, Stärke, Polyvinylpyrrolidin beinhaltet.

5. Biopräparat nach Anspruch 1,
**dadurch gekennzeichnet,**
**dass** es zusätzlich ein Lösungsmittel enthält.

6. Biopräparat nach Anspruch 5,
**dadurch gekennzeichnet,**
**dass** es als Lösungsmittel destilliertes oder abgekochtes Wasser oder physiologische Lösung beinhaltet.

7. Biopräparat nach Ansprüchen 1-6,
**dadurch gekennzeichnet,**
**dass** es lyophil getrocknet ist.

8. Biopräparat nach Ansprüchen 1-6,
**dadurch gekennzeichnet,**
**dass** es granuliert ist.

9. Biopräparat nach Ansprüchen 1-6,
**dadurch gekennzeichnet,**
**dass** es in eine Polymerkapsel geschlossen ist.

10. Biopräparat nach Ansprüchen 1-6,
**dadurch gekennzeichnet,**
**dass** es tablettiert ist.

11. Biopräparat nach Ansprüchen 1-6,
**dadurch gekennzeichnet,**
**dass** es in Form von Zäpfchen oder Suppositorien dargestellt ist.

12. Biopräparat nach Ansprüchen 1-10,
**dadurch gekennzeichnet,**
**dass** es mit Futtermittelkomponenten gemischt ist.

13. Bakteriumstamm *Bacillus subtilis* VKPM B - 8611, der für die Herstellung eines Biopräparats zur Prophylaxe und Behandlung von Infektionserkrankungen und Dysbiose unterschiedlicher Ätiologie des Menschen, des Viehs und des Geflügels verwendet wird.

14. Bakteriumstamm *Bacillus licheniformis* VKPM B - 8610, der für die Herstellung eines Biopräparats zur Prophylaxe und Behandlung von Infektionserkrankungen und Dysbiose unterschiedlicher Ätiologie des Menschen, des Viehs und des Geflügels verwendet wird.

## Claims

1. A biological preparation for medical and veterinary application for the prevention and treatment on infectious diseases and dysbiosis of various etiology of the living microbic mass of the Bacillus subtilis and Bacillus licheniformis strains, and a protective medium,
**characterized in that**
of the Bacillus subtilis strains it contains the strain Bacillus subtilis VKPM B-8611, and of the Bacillus licheniformis strains the strain Bacillus licheniformis VKPM B -8610 in effective amounts.

2. The biological preparation in accordance with claim 1, **characterized in that**
it contains an excipient in addition.

3. The biological preparation in accordance with claim 2, **characterized in that**
as excipient it contains protein, organic polymers, milk, serum, albumin, saccharose, lactose, calcium stearate, glucose, sodium hydrogen carbonate, aluminum hydroxide, methyl cellulose, talcum, fat used in candy making, paraffin, lanolin, cocoa butter, gel of aluminum hydroxide.

4. The biological preparation in accordance with claim 3, **characterized in that**
of organic polymers it contains dextrans, polyglycins, starches, polyvinyl pyrrolidins.

5. The biological preparation in accordance with claim 1, **characterized in that**
in addition it contains a solvent.

6. The biological preparation in accordance with claim 5, **characterized in that**
as the solvent it contains distilled or boiled water or a physiological solution.

7. The biological preparation in accordance with claims 1 to 6,
**characterized in that**
it has been dried lyophilically.

8. The biological preparation in accordance with claims 1 to 6,
**characterized in that**
it is granulated,

9. The biological preparation in accordance with claims 1 to 6,
**characterized in that**
it is enclosed in a polymer capsule.

10. The biological preparation in accordance with claims 1 to 6,
**characterized in that**
it is in tablet form.

11. The biological preparation in accordance with claims 1 to 6,
**characterized in that**
it is presented in the form of capsules or suppositories.

12. The biological preparation in accordance with claims 1 to 6,
**characterized in that**
it is mixed with feed components.

13. The strain Bacillus subtilis VKPM B-8611, which is employed for the prophylaxis and treatment of infectious diseases and dysbiosis of different etiology of humans, livestock and fowl.

14. strain Bacillus licheniformis VKPM B -8610 which is employed for the prophylaxis and treatment of infectious diseases and dysbiosis of different etiology of humans, livestock and fowl.

## Revendications

1. Préparation biologique destinée à une application médicale et vétérinaire et destinée à prévenir ou traiter des maladies infectieuses et des dysbioses de différente étiologie, qui contient une masse microbienne vivante des souches *Bacillus subtilis, Bacillus licheniformis,* et des moyens de protection, **caractérisée en ce qu'**elle contient la souche *Bacillus subtilis* VKPM B - 8611 des souches *Bacillus subtilis* et la souche *Bacillus licheniformis* VKPM B - 8610 des souches *Bacillus licheniformis* dans une quantité efficace.

2. Préparation biologique selon la revendication 1, **caractérisée en ce qu'**elle contient en outre une base.

3. Préparation biologique selon la revendication 2, **caractérisée en ce qu'**elle contient comme base une protéine, un polymère organique, du lait, du sérum, de l'albumine, du saccharose, du lactose, du stéarate de calcium, du glucose, de l'hydrogénocarbonate de sodium, de l'hydroxyde d'aluminium, de la méthylcellulose, du talc, de la graisse de confiserie, de la paraffine, de la lanoline, du beurre de cacao, un gel de l'hydroxyde d'aluminium.

4. Préparation biologique selon la revendication 3, **caractérisée en ce qu'**elle comprend des polymères organiques dextrane, polyglycine, amidon, polyvinylpyrrolidine.

5. Préparation biologique selon la revendication 1, **caractérisée en ce qu'**elle contient en outre un solvant.

6. Préparation biologique selon la revendication 5, **caractérisée en ce qu'**elle contient en tant que solvant de l'eau distillée ou bouillie ou une solution physiologique.

7. Préparation biologique selon les revendications 1 à 6, **caractérisée en ce qu'**elle est séchée par lyophilisation.

8. Préparation biologique selon les revendications 1 à 6, **caractérisée en ce qu'**elle est granulée.

9. Préparation biologique selon les revendications 1 à 6, **caractérisée en ce qu'**elle est enfermée dans une capsule polymère.

10. Préparation biologique selon les revendications 1 à 6, **caractérisée en ce qu'**elle est formée en comprimés.

11. Préparation biologique selon les revendications 1 à 6, **caractérisée en ce qu'**elle est présentée sous la forme de suppositoires.

12. Préparation biologique selon les revendications 1 à 10, **caractérisée en ce qu'**elle est mélangée à des composantes d'aliments pour animaux.

13. Souche bactérienne *Bacillus subtilis* VKPM B - 8611, qui est utilisée pour la préparation d'une préparation biologique destinée à la prophylaxie et au traitement de maladies infectieuses et de dysbioses de différente étiologie chez l'homme, le bétail et la volaille.

14. Souche bactérienne *Bacillus licheniformis* VKPM B - 8610, qui est utilisée pour la préparation d'une préparation biologique destinée à la prophylaxie et au traitement de maladies infectieuses et de dysbioses de différente étiologie chez l'homme, le bétail et la volaille.
